Europäisches Patentamt

European Patent Office

Office européen des brevets

(11) Publication number: **0 072 193**
**B1**

# EUROPEAN PATENT SPECIFICATION

(45) Date of publication of patent specification: **16.10.85**

(51) Int. Cl.⁴: **A 61 B 1/06, G 02 B 6/24**

(21) Application number: **82304105.8**

(22) Date of filing: **04.08.82**

(54) Endoscopes.

(30) Priority: **05.08.81 JP 117200/81 U**

(43) Date of publication of application:
**16.02.83 Bulletin 83/07**

(45) Publication of the grant of the patent:
**16.10.85 Bulletin 85/42**

(84) Designated Contracting States:
**AT BE CH DE FR GB IT LI NL SE**

(56) References cited:
**GB-A-1 365 723**
**GB-A-1 463 350**
**US-A-3 897 134**

(73) Proprietor: **OLYMPUS OPTICAL CO., LTD.**
**43-2, 2-chome, Hatagaya Shibuya-ku**
**Tokyo 151 (JP)**

(72) Inventor: **Hashiguchi, Toshihiko**
**No. 3-15 Seishin 8-chome**
**Sagamihara-shi Kanagawa-ken (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

Courier Press, Leamington Spa, England.

## Description

The invention relates to endoscopes of the type wherein for light guide fibres are coupled to a light guide cable supplying light from an external light source via a light guide connector plug.

An endoscope having an elongate rigid insertion sheath to be inserted into a body cavity and facilitate diagnosis of an affected part or, as required, to perform a therapeutic treatment by using a forceps has recently become extensively used in medical circles. For observation of an affected part by using such a hard structured endoscope, illumination is provided from an external light source through a light guide cable, and this light guide cable is so formed at the end as to be disconnectably coupled with light guide fibres inserted through an insertion sheath to extend from its operational tip to a light guide connector plug attached to the operating handgrip section at the rear of the sheath.

The end surfaces of the light guide fibres in the above mentioned light guide connector plug are ground optically flat after the light guide connecting plug is assembled, so that the illuminating light from the light guide cable supply side may be effectively transmitted. In such case, the end surface of the outer peripheral plug portion around the light guide fibres is also ground down to be flush with the end surfaces of the light guide fibres themselves.

In prior examples, as the peripheral portion of the light guide connector plug is ground, a plating step has been required after the grinding operation. Further, there have been problems introduced because when the connector plug is plated after the grinding, its surface will project by the thickness of the plating film above the end surfaces of the light guide fibres, and dirt may accumulate on the recessed end surfaces of the light guide fibres, and furthermore the illuminating lights will not be as effectively transmitted in any case, because a gap has been introduced. There have been further problems that, due to the difference in thickness caused by this plating film, the connector plug may tend to catch, and therefore, if connection and disconnection are repeated frequently the plating of the light guide connector plug may peel off, the appearance will be impaired and some surface portions may rust when they are disinfected and sterilised.

According to the present invention, a connecting part for light guide fibres connected with a light guide cable leading illuminating light from an external light source is formed of a plurality of members, of which that one constituting an outer peripheral part of the end surfaces of the light guide fibres is formed of a member not plated.

One object of the present invention is to provide an endoscope wherein plating of a light guide connecting part after a grinding operation is made unnecessary, so that the manufacturing steps can be reduced in number, so saving cost, whilst ensuring that the surface of the light guide connector plug is kept flush with the end surfaces of the light guide fibres, to ensure that the end surfaces of the light guide fibres do not tend to collect dirt and the illumination is effectively transmitted, and as the light guide connector plug is formed with a member that does not require to be plated, the appearance will not be impaired, even after frequent connection and disconnection.

According to one aspect the invention comprises an endoscope having a rigid insertion sheath wherein light guide fibres are internally fitted to convey illuminating light supplied from an external light source through a light guide cable coupled at its end to a light guide connector plug fitted to an operating portion of the endoscope at the rear of the sheath, the end surfaces of the light guide fibres mounted within the connector plug being flush with the end surface of the connector plug, and the connector plug being at least partially formed of a plated material, characterised in that the light guide connector plug is formed of a plurality of members, of which that one constituting a peripheral part surrounding the end surfaces of the light guide fibres is an unplated material.

The invention will now be described with reference to the drawings, in which:—

Figure 1 is a side view showing a typical known endoscope of the type having a rigid insertion sheath;

Figure 2 is a magnified sectional view showing part of the rear end operating portion of the known endoscope shown in Figure 1;

Figure 3 is a magnified sectional view showing the peripheral portion of the end of a light guide connector plug provided in the operating portion of this known endoscope;

Figure 4 and others following it show embodiments of the present invention.

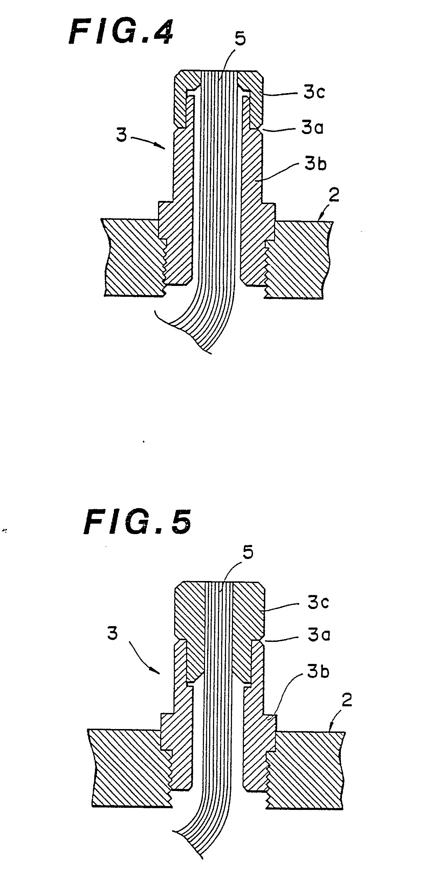

Figure 4 is a sectional view showing the peripheral portion of a light guide connector plug in a first exemplary embodiment of the present invention; and

Figure 5 is a sectional view showing the peripheral portion of a light guide connector plug in a secondary exemplary embodiment of the present invention.

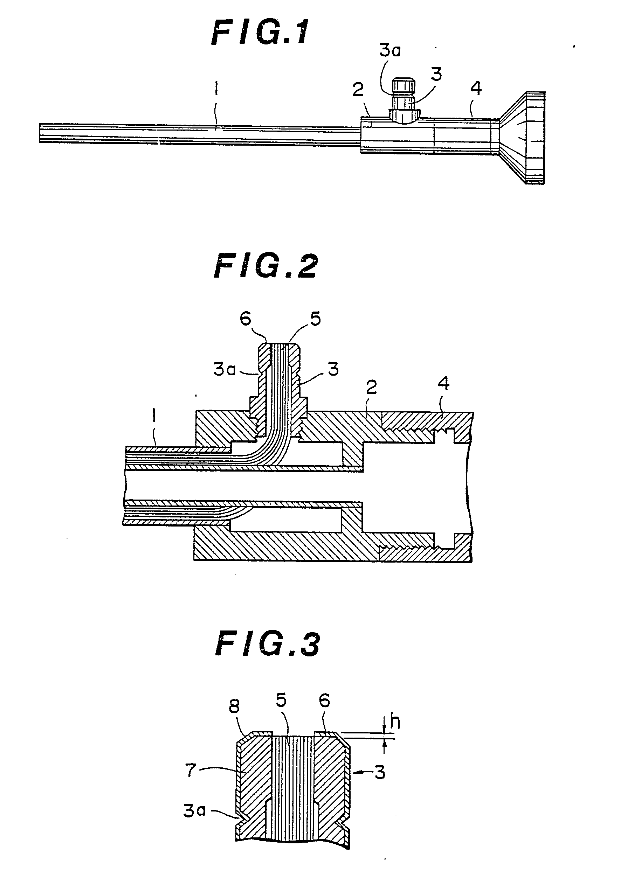

Prior to explaining the embodiments of the present invention, a known example of an endoscope will be described with reference to Figures 1 to 3.

As shown in Figure 1, an endoscope comprises an elongate rigid insertion sheath 1 designed to be inserted into a body cavity, and containing observing and illuminating optical systems, and having an operating portion 2 at the rear end forming a hand grip. A light guide connector plug 3 is formed on one side of the operating portion 2, and an observing eyepiece 4 is formed at the rear end of the operating portion.

A click-stop groove 3a is provided for removably fitting a light guide cable socket for transmitting illuminating light from an external light source (not illustrated), when engaged upon the light guide connector plug 3. Each light guide fibre has an end surface flush with the end of this light guide connector plug 3, transmitting a light down to the tip of the insertion sheath to project illuminating light out from the other end surface of the fibre. The insertion sheath 1, operating portion 2, light guide connector plug 3 and eyepiece 4 are all made of brass, and are plated with chromium or the like after being assembled.

In the step of assembling the light guide connector plug 3 in this known endoscope, as shown in Figure 2, the light guide connector plug 3 is screwed into the operating part, the light guide fibres 5 are bonded to the light guide connector plug with a bonding agent, and then the end surface 6 of the light guide connector plug 3, which plug is formed as a single member, is ground down together with the end surfaces of the light guide fibres 5. The outer surface of the light guide connector plug 3 is then plated, together with the insertion sheath 1, operating portion 2 and eyepiece 4.

However, in this known endoscope as the end surfaces of the light guide fibres 5, which are ground optically flat so that the illuminating light from the light guide cable (not illustrated) may be effectively transmitted between the coupled end surfaces, the end surface 6 of the light guide connector plug 3 surrounding the outer periphery of the light guide fibres 5 will be ground down, and therefore the light guide connecting part 3 will have to be plated after the assembling step. This introduces problems, as the manufacturing steps will be complicated, the plating film 8 will form a different level at a height $h$ above the brass material 7 of the light guide connector plug and the light guide fibres 5, as shown in Figure 3, and therefore dirt may tend to accumulate on the recessed level, and the plating film 8 will be likely to peel off.

The present invention avoids the above-mentioned problems.

Embodiments of the present invention will now be explained in the following with reference to Figure 4 and 5.

A light guide connector plug 3 in the exemplary embodiment shown in Figure 4 is attached to the side of an endoscope operating portion 2 in the same manner as in the above described conventional example. A click-stop groove 3a is formed on this light guide connector plug 3, to be engaged by a light guide cable socket (not illustrated) that is to be removably fitted on the plug. This groove 3a on the light guide connector plug 3 is formed at the junction of two members, of a connector base 3b and an end-cap 3c, the tip of the connector part 3b being reduced in diameter, and pressed and fixed into the end-cap 3c.

The butted parts of the above mentioned connector base 3b and cap 3c are respectively chamfered to form the click-stop groove 3a for fitting the light guide cable (not illustrated). The connector base 3b is of the same brass material as in the conventional example, but is chromium-plated on the surface, and is connected with the operating portions 2 by a screw-threaded part formed at the base end. It is preferable to use a member that is not required to be plated for the cap 3c, so a material of substantially the same colour as the plated surface of the connector base 3b is employed to give a unified appearance. For example, a stainless steel may be used for the cap 3c.

With such formation, in the steps of assembling the endoscope, the parts other than the cap 3c are first respectively plated, the connector base 3b is connected to the operating portion 2, the light guide fibres 5 are inserted through the connector base 3b and then the cap 3c is fitted to bond and fix the light guide fibres 5 and so complete the assembly. Then, the end of the cap 3c and the ends of the light guide fibres 5 are simultaneously ground down. The ground surface obtained by this grinding is a finish not significantly different from the surfaces of the other plated parts in appearance.

Stainless steel is more difficult to thread or bore than brass, so that it is lower in manufacturing cost and therefore more advantageous to form the connector base 3b of a plated brass material and the cap 3c of a stainless steel, as in the above described exemplary embodiment. Further, the reduced diameter stem part of the light guide connector plug 3 will not be visible when the light guide cable is connected, but the large diameter part can always be seen. Therefore, the cap 3c of the light guide connector plug 3 is preferably made of the same plated material as the operation portion 2 to improve the appearance. If a stainless steel is used for the cap 3c, rusting will be prevented during disinfecting and sterilising treatments.

Figure 5 shows a light guide connector plug 3 of a second exemplary embodiment of the present invention. As in the first described embodiment, an endoscope operating portion has a light guide connector plug having a click-stop groove 3a formed between a connector base 3b and an end-cap 3c. The connector base 3b is screwed to the operating portion 2, light guide fibres 5 are inserted through them, and the end cap has a lower portion that is small in diameter and can be pressed into the connector base 3b. The light guide fibres 5 are bonded and fixed, and then the end-cap 3c and light guide fibre 5 are simultaneously ground. The difference from the first described embodiment of the invention is that in this case the cap 3c is pressed into the connector base 3b.

The same materials as described for the first embodiment can be used for the connector base 3b and cap 3c in this second exemplary embodiment.

In the above described embodiments, the connector base 3b and cap 3c in the light guide connecting part 3 are not necessarily fitted together by pressing, but can be formed so as to be fixed together by screwing, for example.

Further, in the above described embodiments, the light guide connector plug 3 is formed of only two members, but it will be readily apparent that it can be formed of three or more members.

Different working modes can be utilised in variations of the described embodiments without deviating from the scope of the present invention, which is defined by the claims.

## Claims

1. An endoscope having a rigid insertion sheath (1) wherein light guide fibres (5) are internally fitted to convey illuminating light supplied from an external light source through a light guide cable coupled at its end to a light guide connector plug (3) fitted to an operating portion (2) of the endoscope at the rear of the sheath, the end surfaces of the light guide fibres mounted within the connector plug being flush with the end surface (6) of the connector plug, and the connector plug being at least partially formed of a plated material, characterised in that the light guide connector plug is formed of a plurality of members (3b, 3c), of which that one (3c) constituting a peripheral part surrounding the end surfaces of the light guide fibres is an unplated material.

2. An endoscope according to Claim 1, characterised in that said unplated material has substantially the same colour as other parts (3b, 2) of the endoscope, which are plated parts.

3. An endoscope according to Claim 1, characterised in that said unplated material is stainless steel.

## Revendications

1. Endoscope muni d'une gaine d'insertion rigide (1) dans laquelle des fibres de guidage de lumière (5) sont montées intérieurement pour transporter la lumière d'illumination provenant d'une source de lumière extérieure par un câble de guidage de lumière couplé, par son extrémité, à une fiche de connecteur de guide de lumière (3) s'adaptant sur une partie de fonctionnement (2) de l'endoscope à l'arrière de la gaine, les surfaces d'extrémité des fibres de guidage de lumière montées dans la fiche de connecteur, venant à ras de la surface d'extrémité (6) de la fiche de connecteur, et la fiche de connecteur étant, au moins partiellement, réalisée dans un matériau plaqué, endoscope caractérisé en ce que la fiche de connecteur de guide de lumière est constituée d'un certain nombre d'éléments (3b, 3c) parmi lesquels celui (3c) qui constitue la partie périphérique entourant les surfaces d'extrémité des fibres de guidage de lumière, est en matériau non plaqué.

2. Endoscope selon la revendication 1, caractérisé en ce que le matériau non plaqué présente exactement la même couleur que les autres parties (3b, 2) de l'endoscope, qui sont des parties plaquées.

3. Endoscope selon la revendication 1, caractérisé en ce que le matériau non plaqué est de l'acier inoxydable.

## Patentansprüche

1. Endoskop mit einem unbiegsamen Einführungsschlauch (1), bei dem Lichtleiterfasern (5) derart im Innern angebracht sind, daß sie von einer äußeren Lichtquelle geliefertes Beleuchtungslicht durch ein Lichtleiterkabel leiten, welches endseitig mit einem an einem Bedienungsteil (2) des Endoskops am hinteren Ende des Schlauchs angebrachten Lichtleiteranschlußstecker (3) gekoppelt ist, wobei die Stirnflächen der im Innern des Anschlußsteckers angebrachten Lichtleiterfasern in einer Ebene mit der Stirnfläche (6) des Anschlußsteckers liegen, und der Anschlußstecker wenigstens teilweise aus plattiertem Werkstoff geformt ist, dadurch gekennzeichnet, daß der Lichtleiteranschlußstecker aus mehreren Teilen (3b, 3c) besteht, von denen dasjenige (3c), das ein die Stirnfläche der Lichtleiterfasern umgebendes Umfangsteil bildet, ein unplattierter Werkstoff ist.

2. Endoskop nach Anspruch 1, dadurch gekennzeichnet, daß der unplattierte Werkstoff im wesentlichen die gleiche Farbe hat wie andere Teile (3b, 2) des Endoskops, die plattierte Teile sind.

3. Endoskop nach Anspruch 1, dadurch gekennzeichnet, daß der unplattierte Werkstoff rostfreier Stahl ist.

0 072 193

# FIG.1

# FIG.2

# FIG.3

1

# FIG.4

5
3c
3
3a
3b
2

# FIG.5

5
3c
3a
3
3b
2